# EUROPEAN PATENT SPECIFICATION

(11) **EP 1 332 755 B1**
(45) Date of publication and mention of the grant of the patent: **29.09.2010**
(21) Application number: 01919099.0
(22) Date of filing: 28.02.2001
(51) Int. Cl.: A61K 9/127, A61K 31/337

(54) **Paclitaxel Liposome Composition For Treatment of Cancer and Preparation Thereof**
Liposomzusammensetzungen, Paclitaxel enthaltend, zur Behandlung von Krebs und deren Zubereitung
Compositions de liposomes contenant du paclitaxel pour le traitement du cancer et leur préparation

(30) Priority: 19.10.2000 CN 00119039
(43) Date of publication of application: 06.08.2003
(73) Proprietor: Nanjing Zhenzhong Bioengineering Company Ltd., JiangSu 210061 (CN)
(72) Inventor: WENG, Guoying, JiangSu 210061 (CN); ZHOU, Wei, Nanjing 210009 (CN); CHENG, Guang, JiangSu 210061 (CN); CHENG, Peiyuan, JiangSu 210061 (CN)
(74) Representative: Flaccus, Rolf-Dieter
(86) International application number: PCT/CN2001/000309
(87) International publication number: WO 2002/032399

(56) References cited:
- WO-A-94/26254
- CN-A- 1 148 957
- US-A- 5 576 017
- US-A- 5 648 090
- US-A- 5 891 467
- US-A- 6 090 955
- SHIEH M-F ET AL: "LIPOSOMAL DELIVERY SYSTEM FOR TAXOL" JOURNAL OF FERMENTATION AND BIOENGINEERING, SOCIETY OF FERMENTATION TECHNOLOGY, JP, vol. 83, no. 1, 1997, pages 87-90, XP000965160 ISSN: 0922-338X
- JIALIN YAN ET AL: 'Prepartion of taxol liposome and their study in suppressing' PHARMACOLOGICAL BIOTECHNOLOGY vol. 3, no. 3, 1996, pages 154 - 157, XP002971771

## Description

### Field of the Invention

The present invention relates to a pharmaceutical composition for use in the treatment of cancer, especially relates to a paclitaxel liposome composition for use in the treatment of cancer and method of preparation thereof.

### Description of the Related Art

Paclitaxel is an anticarcinogen found by the National Cancer Institute, USA through screening thousands of plants in the 1960s. It is generally used for treatment of oophoroma, breast carcinoma and non-small-cell lung carcinoma. Because paclitaxel is difficult to be dissolved in water and many officinal solvents, all of the paclitaxel injections provided presently in the domestic and overseas market were prepared with the complex dissolvent of polyoxyethylated castor oil and anhydrous ethanol. The polyoxyethylated castor oil in the complex dissolvent will result in the release of histamine when degraded in body, and thus may cause serious hyperallergic reaction/pleoergy.

A paclitaxel injection (trade name is Taxol) is registered in China by Bristol-Myers Squibb Company, USA, and it was pointed out in the specification that "all patients who receive the Taxol should be given in advance corticosteroid (such as dexamethasone), diphenhydramine and H₂ receptor antagonist (such as cimitidine, ranitidine) in order to prevent the occurrence of serious hyperallergic reaction." And it also said that "precipitation (crystal) may appear when diluted with physiological saline or 5% glucose before injection. So it is necessary to be filtrated with 0.22 µm millipore filter to assure the safety in administration." Also paclitaxel itself has haematics toxicity, bone marrow depression, leukopenia, thrombocytopenia, anemia and other toxicities. Therefore, change of paclitaxel's dosage form are demanded urgently to increase its solubility, improve its stability and clinical compliance, and avoid the allergic reaction caused by the above complex dissolvent and other toxic side effect.

Practicable progress was achieved from the basic researches of paclitaxel liposome in the early 1990s and the recent preclinical study. The experiments indicated that the paclitaxel liposome preparation had similar therapeutic effectiveness with reduced toxicity and improved tolerance compared to the paclitaxel injection preparation. SharmaA reported a paclitaxel liposome (code name TTL) made from three synthetic phosphatides (Int. J. Cancer 1997, 71, 103-107) with better stability than the paclitaxel liposome (code name ETL) made from single lecithin. But it did not say how much the difference of stability was between the two preparations, and only mentioned that the ETL preparation maintained its stability for 24 hours after dissolved in water and that the solvent used was benzene or butanol. Benzene has much toxicity when used as solvent.

In US patent No. 5,415,869 (1995), it was mentioned that aggregation occurred in the paclitaxel liposome preparation made from single lecithin. Thus electronegative or electropositive ingredient such as phosphatidyl glycerol (PG) was introduced. The ratio of lecithin to PG adopted by the patent was 9:1 or 3:7 while chloroform was used as solvent, the paclitaxel liposome did not aggregation. But when the ratio was 5:5 the paclitaxel liposome crystal precipitated. PG is difficult to obtain due to its low content in lecithin. And there are only expensive products of PG of reagent grade overseas. Also chloroform has too much toxicity when used as solvent.

US 5 891 467 B discloses multivesicular liposomes comprising also neutral lipids (e.g. triolein) ;
Shieh M-F et al. , J. Fermentation and Bioengineering 83(1),pages 87-90, 1997 discloses taxol liposomes comprising lecithin, dimirystoylphosphatidylglycerol, cholesterol and D,L-α-tocopherol.

### SUMMARY OF THE PRESENT INVENTION

One object of the present invention is to provide a paclitaxel liposome composition for treatment of cancer without polyoxyethylated castor oil.

The second object of the present invention is to provide a paclitaxel liposome composition for treatment of cancer, which has good colliquefaction with the water soluble transfusion and can be infused directly into 5% glucose solution for intravenous drip after shaking, and has reduced toxicity and can avoid the hyperallergic reaction caused by the complex dissolvent and has improved stability and relatively lower cost.

The third object of the present invention is to provide a method of preparation of paclitaxel liposome composition for treatment of cancer without polyoxyethylated castor oil.

The fourth object of the present invention is to provide a method of preparation of paclitaxel liposome composition for treatment of cancer with improved water solubility and stability using liposome imbedding technique.

These and other objects will be further described and represented through the following detailed illustration.

### DETAILED DESCRIPTION OF THE INVENTION

In the present invention, the paclitaxel liposome composition for treatment of cancer consists substantially of the following materials by weight:
Paclitaxel 2-5 parts, Phosphatide 20-200 parts, Cholesterol 2-30 parts,
Amino acids 0.3-4 parts, Lyophilized excipient 10-75 parts.

Furthermore, the paclitaxel liposome composition for treatment of cancer of the present invention consists substantially of the following materials by weight:
Paclitaxel 3-5 parts, Phosphatide 40-160 parts, Cholesterol 5-25 parts,
Amino acids 0.8-3 parts, Lyophilized excipient 20-65 parts.

The paclitaxel liposome composition for treatment of cancer of the present invention may also consist substantially of the following materials by weight:
Paclitaxel 3-5 parts, Phosphatide 60-120 parts, Cholesterol 8-20 parts,
Amino acids 1.0-2 parts, Lyophilized excipient 30-60 parts.

The paclitaxel liposome composition for treatment of cancer of the present invention does not contain polyoxyethylated castor oil.

Furthermore, in the paclitaxel liposome composition for treatment of cancer of the present invention, the said lyophilized excipient is mannitol, sucrose, glucose, or lactose which can be solid suited for injection. The said amino acid can be lysine, threonine or methionine. The said phosphatide is egg yolk lecithin or soy bean lecithin for injection.

If necessary, the composition of the present invention can also be added or together with other anti-tumor drugs. The suitable anti-tumor drug and adjuvant may be diphenhydramine, cimitidine, niacinamide, VB₆, VB₁, or may also be bear gall powder, rhodiola root, ginseng, American ginseng, cordyceps, ganoderma lucidum and hsueh-lien-hua, etc.

The paclitaxel liposome composition for treatment of cancer of the present invention can be prepared by the following method, and includes substantially the following materials by weight:
Paclitaxel 2-5 parts, Phosphatide 20-200 parts, Cholesterol 2-30 parts,
Amino acids 0.3-4 parts, Lyophilized excipient 10-75 parts.

Paclitaxel, phosphatide, cholesterol are agitated and dissolved successively in isopropanol or ethanol using the above ratios to obtain a clear solution. Then the solution is placed in a constant temperature water bath with a temperature of 50-60°C. After the solvent is removed with a rotatory evaporator under reduced pressure, a membrane is formed therefrom. The aqueous solution of amino acids and lyophilized excipient dissolved in the above ratios is introduced. And then hydration, sonication or homogenization is conducted to attain the liposome size above 0.1 µ m, preferably 0.2-5 µ m. After sterilization filtration, the gain is subpackaged into containers such as ampoules or vials etc., and lyophilized to obtain white lumpy paclitaxel liposome. Nitrogen, helium or argon gas can be aerated when sealing or capping.

Furthermore, the paclitaxel liposome composition for treatment of cancer of the present invention can be prepared by the following method, and includes substantially the following materials by weight:
Paclitaxel 3-5 parts, Phosphatide 40-160 parts, Cholesterol 5-25 parts,
Amino acids 0.8-3 parts, Lyophilized excipient 20-65 parts.

Paclitaxel, phosphatide, cholesterol are agitated and dissolved successively in isopropanol or ethanol using the above ratios to obtain a clear solution. Then the solution is placed in a constant temperature water bath with a temperature of 50-60°C. After the solvent is removed with a rotatory evaporator under reduced pressure, a membrane is formed therefrom. The aqueous solution of amino acids and lyophilized excipient dissolved in the above ratios is introduced. And then hydration, sonication or homogenization is conducted to attain the liposome size 0.1-5 µm. After sterilization filtration, the gain is subpackaged into containers such as ampoules or vials etc., and lyophilized to obtain white lumpy paclitaxel liposome. Nitrogen, helium or argon gas can be aerated when sealing or capping.

The paclitaxel liposome composition for treatment of cancer of the present invention can also be prepared by the following method, and includes substantially the following materials by weight:
Paclitaxel 3-5 parts, Phosphatide 60-120 parts, Cholesterol 8-20 parts,
Amino acids 1.0-2 parts, Lyophilized excipient 30-60 parts.

Paclitaxel, phosphatide, cholesterol are agitated and dissolved successively in isopropanol or ethanol in the above ratios to obtain a clear solution. Then the solution is placed in a constant temperature water bath with a temperature of 50-60°C. After the solvent is removed with a rotatory evaporator under reduced pressure, a membrane is formed therefrom. The aqueous solution of amino acids and lyophilized excipient dissolved in the above ratios is introduced. And then hydration, sonication or homogenization is conducted to attain the liposome size above 0.1 µm. After sterilization filtration, the gain is subpackaged into containers such as ampoules or vials etc., and lyophilized to obtain white lumpy paclitaxel liposome. Nitrogen, helium or argon gas can be aerated when sealing or capping.

In the above method, the said lyophilized excipient is mannitol, sucrose, glucose, or lactose. The said amino acid can be lysine, threonine or methionine. The said phosphatide is egg yolk lecithin or soy bean lecithin for injection.

The products of the present invention do not contain polyoxyethylated castor oil and substitute the toxic dissolvent and expensive adjuvant with nontoxic dissolvent and easily obtained adjuvant, and can be executed in industry scale. So they have advantages of low toxicity, good water solubility and better stability, etc. They can be infused directly into 5% glucose solution for intravenous drip after shaking and avoid the hyperallergic reaction caused by the complex dissolvent, and have relatively lower cost.

The paclitaxel liposome preparation prepared according to the present invention has similar anti-cancer effectiveness to the commercial paclitaxel injection, as shown in table 1.

**Table 1. The inhibitory effect of paclitaxel liposome for injection and commercial paclitaxel injection on S-180**

| Group | Dosage (mg/kg) | Number of animals | | Tumour weight (g) | Inhibition ratio (%) |
|---|---|---|---|---|---|
| | | Before administration | After administration | | |
| Paclitaxel liposome | 20.0 | 10 | 10 | 0.826±0.204** | 48.79 |
| Paclitaxel liposome | 14.0 | 10 | 10 | 0.896±0.293** | 44.44 |
| Paclitaxelliposome | 9.8 | 10 | 10 | 1.114±0.425 | 30.89 |
| Paclitaxel injection | 14.0 | 10 | 10 | 0.860±0.177** | 46.65 |
| Paclitaxel injection | 9.8 | 10 | 9 | 1.180±0.299 | 26.80 |
| Control | | 10 | 10 | 1.612±0.705 | |

| | | | | | |
|---|---|---|---|---|---|
| ** P<0.01, compared with the control group. | | | | | |

In the present invention, the single lecithin together with cholesterol and amino acids is used as the stabilizing agent. Amino acids are amphoteric material, which can show electrification at given pHs such as in the present technical scheme. So this will prevent aggregation and precipitations of taxol crystal. The method uses easily obtained material so that the cost is low compared to the reported methods, in which electronegative ingredient such as PG or electropositive ingredient were added. And the obtained paclitaxel liposome has better stability than that only with single lecithin. Aggregation does not occur and crystal does not precipitate in the obtained paclitaxel liposome when standing after dissolved with water (table 2).

**Table 2. Data of paclitaxel liposome's stability at low temperature (2-10°C)**

| Lot number | Time (month) | Appearance | Microscopic examination (1 × 1600) |
|---|---|---|---|
| No. 1 | 0 | Loose offwhite lump | No crystal after dissolved, no aggregation |
| | 1 | Loose offwhite lump | No crystal after dissolved, no aggregation |
| | 6 | Loose offwhite lump | No crystal after dissolved, no aggregation |
| | 12 | Loose offwhite lump | No crystal after dissolved, no aggregation |
| No. 2 | 0 | Loose offwhite lump | No crystal after dissolved, no aggregation |
| | 1 | Loose offwhite lump | No crystal after dissolved, no aggregation |
| | 6 | Loose offwhite lump | No crystal after dissolved, no aggregation |
| | 12 | Loose offwhite lump | No crystal after dissolved, no aggregation |

The paclitaxel liposome preparation according to the present invention has better colliquefaction with the glucose for injection and physiological saline. The content of paclitaxel in the obtained preparation is 4-6 mg/ml, which is a suitable concentration for clinical administration and can be used in clinical therapy with the same amount as the commercial paclitaxel injection. According to body surface area, the dosage is 175 mg/m² intravenous drip.

In the present invention, all materials and adjuvants can be purchased in the market.

The present invention will be illustrated further with several examples. But these examples are used for illustration of the present invention, and do not limit the scope of the present invention.

If not being pointed out specially, all parts or measurements are weight units based on the total weight.

### Example 1

In aseptic condition, paclitaxel for injection (2.5g), refined egg yolk lecithin for injection (30g) and cholesterol (2.7g) were introduced into a round-bottomed flask. And proper amount of isopropanol (about 300ml) was added to make the mixture dissolved completely as clear solution. Then underpressure drying was conducted with a rotary evaporator in a constant temperature water bath (50°C) to form a membrane. 5% mannitol solution containing 2.8g lysine was added to dissolve the membrane, and a sonifier was used for ultrasonic pulverization. After being filtrated with 0.22 µm filter membrane to degerm, the solution was subpackaged into ampoules (or vials) so that 25mg paclitaxel was contained in each bottle. Lyophilization was conducted followed by sealing in inert gases to obtain white lumpy paclitaxel liposome preparation.

### Example 2

In aseptic condition, paclitaxel for injection (5.0g), refined soy bean lecithin for injection (72g) and cholesterol (6g) were introduced into a round-bottomed flask. And proper amount of ethanol (about 800ml) was added to make the mixture dissolved completely as clear solution. Then underpressure drying was conducted with a rotary evaporator in a constant temperature water bath (50°C) to form a membrane. 5% mannitol solution containing 3.4g lysine was added to dissolve the membrane, and a high-pressure refiner was used for homogenization. After being filtrated with 0.22 µ m filter membrane to degerm, the solution was subpackaged into ampoules (or vials) so that 30mg paclitaxel was contained in each bottle. Lyophilization was conducted followed by sealing in inert gases to obtain white lumpy paclitaxel liposome preparation.

### Example 3

In aseptic condition, paclitaxel for injection (2.5g), refined soy bean lecithin for injection (30g) and cholesterol (2.7g) were introduced into a round-bottomed flask. And proper amount of isopropanol (about 400ml) was added to make the mixture dissolved completely as clear solution. Then underpressure drying was conducted with a rotary evaporator in a constant temperature water bath (50°C) to form a membrane. 7.5% sucrose solution containing 2.0g methionine was added to dissolve the membrane, and a sonifier was used for ultrasonic pulverization. After being filtrated with 0.22 µm filter membrane to degerm, the solution was subpackaged into ampoules (or vials) so that 20mg paclitaxel was contained in each bottle. Lyophilization was conducted followed by sealing in inert gases to obtain white lumpy paclitaxel liposome preparation.

### Example 4

In aseptic condition, paclitaxel for injection (5.0g), refined egg yolk lecithin for injection (72g) and cholesterol (6g) were introduced into a round-bottomed flask. And proper amount of ethanol (about 1500ml) was added to make the mixture dissolved completely as clear solution. Then underpressure drying was conducted with a rotary evaporator in a constant temperature water bath (50°C) to form a membrane. 7.5% mannitol solution containing 3.5g threonine was added to dissolve the membrane, and a high-pressure refiner was used for homogenization. After being filtrated with 0.22 µm filter membrane to degerm, the solution was subpackaged into ampoules (or vials) so that 30mg paclitaxel was contained in each bottle. Lyophilization was conducted followed by sealing in inert gases to obtain white lumpy paclitaxel liposome preparation.

### Example 5

In aseptic condition, paclitaxel for injection (5.0g), refined egg yolk lecithin for injection (65g) and cholesterol (6.5g) were introduced into a round-bottomed flask. And proper amount of ethanol (about 1500ml) was added to make the mixture dissolved completely as clear solution. Then underpressure drying was conducted with a rotary evaporator in a constant temperature water bath (50°C) to form a membrane. 7.5% lactose solution containing 3.8g lysine was added to dissolve the membrane, and a high-pressure refiner was used for homogenization. After beingfiltrated with 0.22 µm filter membrane to degerm, the solution was subpackaged into ampoules (or vials) so that 20mg paclitaxel was contained in each bottle. Lyophilization was conducted followed by sealing in inert gases to obtain white lumpy paclitaxel liposome preparation.

### Example 6

In aseptic condition, paclitaxel for injection (2.0g), refined egg yolk lecithin for injection (42g) and cholesterol (4g) were introduced into a round-bottomed flask. And proper amount of ethanol (about 260ml) was added to make the mixture dissolved completely as clear solution. Then underpressure drying was conducted with a rotary evaporator in a constant temperature water bath (60°C) to form a membrane. 5% mannitol solution (about 400ml) containing 2.0g lysine was added to dissolve the membrane, and a high-pressure refiner was used for homogenization. After being filtrated with 0.22 µm filter membrane to degerm, the solution was subpackaged into ampoules (or vials). Lyophilization was conducted followed by sealing in inert gases to obtain white lumpy paclitaxel liposome preparation.

### Example 7

In aseptic condition, paclitaxel for injection (2.8g), refined egg yolk lecithin for injection (60g) and cholesterol (4g) were introduced into a round-bottomed flask. And proper amount of ethanol (about 350ml) was added to make the mixture dissolved completely as clear solution. Then underpressure drying was conducted with a rotary evaporator in a constant temperature water bath (55°C) to form a membrane. 5% mannitol solution (about 300ml) containing 2.4g threonine was added to dissolve the membrane, and a high-pressure refiner was used for homogenization. After being filtrated with 0.22 µm filter membrane to degerm, the solution was subpackaged into ampoules (or vials). Lyophilization was conducted followed by sealing in inert gases to obtain white lumpy paclitaxel liposome preparation.

### Example 8

In aseptic condition, paclitaxel for injection (3.5g), refined soy bean lecithin for injection (100g) and cholesterol (4.5g) were introduced into a round-bottomed flask. And proper amount of isopropanol (about 1000ml) was added to make the mixture dissolved completely as clear solution. Then underpressure drying was conducted with a rotary evaporator in a constant temperature water bath (58°C) to form a membrane. 5% sucrose solution (about 500ml) containing 3.1g methionine was added to dissolve the membrane, and a high-pressure refiner was used for homogenization. After being filtrated with 0.22 µm filter membrane to degerm, the solution was subpackaged into ampoules (or vials). Lyophilization was conducted followed by sealing in inert gases to obtain white lumpy paclitaxel liposome preparation.

### Example 9

In aseptic condition, paclitaxel for injection (4.5g), refined soy bean lecithin for injection (122g) and cholesterol (8g) were introduced into a round-bottomed flask. And proper amount of isopropanol (about 1200ml) was added to make the mixture dissolved completely as clear solution. Then underpressure drying was conducted with a rotary evaporator in a constant temperature water bath (52°C) to form a membrane. 5% mannitol solution (about 1000ml) containing 3.1g threonine was added to dissolve the membrane, and a high-pressure refiner was used for homogenization. After being filtrated with 0.22 µm filter membrane to degerm, the solution was subpackaged into ampoules (or vials). Lyophilization was conducted followed by sealing in inert gases to obtain white lumpy paclitaxel liposome preparation.

### Example 10

In aseptic condition, paclitaxel for injection (5.0g), refined egg yolk lecithin for injection (100g) and cholesterol (15g) were introduced into a round-bottomed flask. And proper amount of ethanol (about 1200ml) was added to make the mixture dissolved completely as clear solution. Then underpressure drying was conducted with a rotary evaporator in a constant temperature water bath (53°C) to form a membrane. 5% mannitol solution (about 1000ml) containing 3.8g lysine was added to dissolve the membrane, and a high-pressure refiner was used for homogenization. After being filtrated with 0.22 µ m filter membrane to degerm, the solution was subpackaged into ampoules (or vials). Lyophilization was conducted followed by sealing in inert gases to obtain white lumpy paclitaxel liposome preparation.

### Example 11

In aseptic condition, paclitaxel for injection (5.0g), refined egg yolk lecithin for injection (120g) and cholesterol (21g) were introduced into a round-bottomed flask. And proper amount of ethanol (about 1500ml) was added to make the mixture dissolved completely as clear solution. Then underpressure drying was conducted with a rotary evaporator in a constant temperature water bath (59°C) to form a membrane. 5% glucose solution (about 1400ml) containing 2.4g threonine was added to dissolve the membrane, and a high-pressure refiner was used for homogenization. After being filtrated with 0.22 µm filter membrane to degerm, the solution was subpackaged into ampoules (or vials) so that 30mg paclitaxel was contained in each bottle. Lyophilization was conducted followed by sealing in inert gases to obtain white lumpy paclitaxel liposome preparation.

### Example 12

In aseptic condition, paclitaxel for injection (4.0g), refined egg yolk lecithin for injection (120g) and cholesterol (18g) were introduced into a round-bottomed flask. And proper amount of ethanol (about 1200ml) was added to make the mixture dissolved completely as clear solution. Then underpressure drying was conducted with a rotary evaporator in a constant temperature water bath (52°C) to form a membrane. 5% lactose solution (about 1000ml) containing 3.0g methionine was added to dissolve the membrane, and a high-pressure refiner was used for homogenization. After being filtrated with 0.22 µm filter membrane to degerm, the solution was subpackaged into ampoules (or vials) so that 25mg paclitaxel was contained in each bottle. Lyophilization was conducted followed by sealing in inert gases to obtain white lumpy paclitaxel liposome preparation.

### Example 13

In aseptic condition, paclitaxel for injection (5.0g), soy bean lecithin (200g) and cholesterol (30g) were introduced into a round-bottomed flask. And proper amount of ethanol (about 2000ml) was added to make the mixture dissolved completely as clear solution. Then underpressure drying was conducted with a rotary evaporator in a constant temperature water bath (57°C) to form a membrane. 5% glucose solution (about 1400ml) containing 4.0g lysine was added to dissolve the membrane, and a high-pressure refiner was used for homogenization. After being filtrated with 0.22 µm filter membrane to degerm, the solution was subpackaged into ampoules (or vials) so that 20mg paclitaxel was contained in each bottle. Lyophilization was conducted followed by sealing in inert gases to obtain white lumpy paclitaxel liposome preparation.

### Example 14

In aseptic condition, paclitaxel for injection (3.0g), refined egg yolk lecithin for injection (80g) and cholesterol (5.0g) were introduced into a round-bottomed flask. And proper amount of isopropanol (about 700ml) was added to make the mixture dissolved completely as clear solution. Then underpressure drying was conducted with a rotary evaporator in a constant temperature water bath (55°C) to form a membrane. 5% mannitol solution (about 1000ml) containing 2.4g lysine was added to dissolve the membrane, and a high-pressure refiner was used for homogenization. After being filtrated with 0.22 µm filter membrane to degerm, the solution was subpackaged into ampoules (or vials) so that 15mg paclitaxel was contained in each bottle. Lyophilization was conducted followed by sealing in inert gases to obtain white lumpy paclitaxel liposome preparation.

## Claims

1. A paclitaxel liposome composition for use in the treatment of cancer, wherein the composition consists substantially of the following materials by weight:
Paclitaxel 2-5 parts, Phosphatide 20-200 parts, Cholesterol 2-30 parts,
Amino acids 0.3-4 parts, Lyophilized excipient 10-75 parts.

2. The paclitaxel liposome composition for use in the treatment of cancer according to Claim 1, wherein the composition consists essentially of the following materials by weight:
Paclitaxel 3-5 parts, Phosphatide 40-160 parts, Cholesterol 5-25 parts,
Amino acids 0.8-3 parts, Lyophilized excipient 20-65 parts.

3. The paclitaxel liposome composition for use in the treatment of cancer according to Claim 1, wherein the composition consists essentially of the following materials by weight:
Paclitaxel 3-5 parts, Phosphatide 60-120 parts, Cholesterol 8-20 parts,
Amino acids 1.0-2 parts, Lyophilized excipient 30-60 parts.

4. The paclitaxel liposome composition for use in the treatment of cancer according to Claims 1 to 3, wherein the composition does not contain polyoxyethylated castor oil.

5. The paclitaxel liposome composition for use in the treatment of cancer according to Claims 1 to 4, wherein the said lyophilized excipient is mannitol, sucrose, glucose, or lactose.

6. The paclitaxel liposome composition for use in the treatment of cancer according to Claims 1 to 4, wherein the said amino acid can be lysine, threonine or methionine.

7. The paclitaxel liposome composition for use in the treatment of cancer according to Claims 1 to 4, wherein the said phospatide is egg yolk lecithin or soy bean lecithin for injection.

8. A method of preparing the paclitaxel liposome composition for use in the treatment of cancer according to Claims 1 to 4, wherein the method comprises the following steps:
- 2-5 parts by weight of paclitaxel , 20-200 parts by weight of phosphatide, 2-30 parts by weight of cholesterol are agitated and dissolved successively in isopropanol or ethanol to obtain a clear solution;
- then the solution is placed in a constant temperature water bath with a temperature of 50-60°C
- after the solvent is removed with a rotatory evaporator under reduced pressure, a membrane is formed therefrom;
- the aqueous solution of 0.3-4 parts by weight of amino acids and 10-75 parts by weight of lyophilized excipient is introduced;
- and then hydration, sonication or homogenization is conducted to attain the liposome size above 0.1 µm;
- after sterilization filtration, the gain is subpackaged into containers, and
- nitrogen, helium or argon gas is aerated after being lyophilized to obtain paclitaxel liposome preparation.

9. The method for preparing the paclitaxel liposome composition for use in the treatment of cancer according to Claims 1 to 4, wherein the method comprises the following steps:
- 3-5 parts by weight of paclitaxel , 40-160 parts by weight of phosphatide, 5-25 parts by weight of cholesterol are agitated and dissolved successively in isopropanol or ethanol to obtain a clear solution;
- then the solution is placed in a constant temperature water bath with a temperature of 50-60°C
- after the solvent is removed with a rotatory evaporator under reduced pressure, a membrane is formed therefrom;
- the aqueous solution of 0.8-3 parts by weight of amino acids and 20-65 parts by weight of lyophilized excipient is introduced;
- and then hydration, sonication or homogenization is conducted;
- after sterilization filtration, the gain is subpackaged into containers, and
- nitrogen, helium or argon gas is aerated after being lyophilized to obtain paclitaxel liposome preparation.

10. The method for preparing the paclitaxel liposome composition for use in the treatment of cancer according to Claims 1 to 4, wherein the method comprises the following steps:
- 3-5 parts by weight of paclitaxel , 60-120 parts by weight of phosphatide, 8-20 parts by weight of cholesterol are agitated and dissolved successively in isopropanol or ethanol to obtain a clear solution;
- then the solution is placed in a constant temperature water bath with a temperature of 50-60°C;
- after the solvent is removed with a rotatory evaporator under reduced pressure, a membrane is formed therefrom;
- the aqueous solution of 1.0-2 parts by weight of amino acids and 30-60 parts by weight of lyophilized excipient is introduced;
- and then hydration, sonication or homogenization is conducted;
- after sterilization filtration, the gain is subpackaged into containers; and
- nitrogen, helium or argon gas is aerated after being lyophilized to obtain paclitaxel liposome preparation.

11. The method for preparing a paclitaxel liposome composition for use in the treatment of cancer according to Claims 8 to 10, wherein the said lyophilized excipient is mannitol, sucrose, glucose, or lactose.

12. The method for preparing a paclitaxel liposome composition for use in the treatment of cancer according to Claims 8 to 10, wherein the said amino acid can be lysine, threonine or methionine.

13. The method for preparing a paclitaxel liposome composition for use in the treatment of cancer according to Claims 8 to 10, wherein the said phospatide is egg yolk lecithin or soy bean lecithin for injection.

## Patentansprüche

1. Paclitaxel-Liposom-Zusammensetzung zur Verwendung in der Behandlung von Krebs, wobei die Zusammensetzung im Wesentlichen aus den folgenden Stoffen, in Gewichtsteilen, besteht:
Paclitaxel 2-5 Teile, Phosphatid 20-200 Teile,
Cholesterin 2-30 Teile, Aminosäuren 0,3-4 Teile,
gefriergetrockneter Hilfsstoff 10-75 Teile.

2. Paclitaxel-Liposom-Zusammensetzung zur Verwendung in der Behandlung von Krebs nach Anspruch 1, wobei die Zusammensetzung im Wesentlichen aus den folgenden Stoffen, in Gewichtsteilen, besteht:
Paclitaxel 3-5 Teile, Phosphatid 40-160 Teile,
Cholesterin 5-25 Teile, Aminosäuren 0,8-3 Teile,
gefriergetrockneter Hilfsstoff 20-65 Teile.

3. Paclitaxel-Liposom-Zusammensetzung zur Verwendung in der Behandlung von Krebs nach Anspruch 1, wobei die Zusammensetzung im Wesentlichen aus den folgenden Stoffen, in Gewichtsteilen, besteht:
Paclitaxel 3-5 Teile, Phosphatid 60-120 Teile,
Cholesterin 8-20 Teile, Aminosäuren 1,0-2 Teile,
gefriergetrockneter Hilfsstoff 30-60 Teile.

4. Paclitaxel-Liposom-Zusammensetzung zur Verwendung in der Behandlung von Krebs nach den Ansprüchen 1 bis 3, wobei die Zusammensetzung kein polyoxyethyliertes Rizinusöl enthält.

5. Paclitaxel-Liposom-Zusammensetzung zur Verwendung in der Behandlung von Krebs nach den Ansprüchen 1 bis 4, bei welcher es sich bei dem gefriergetrockneten Hilfsstoff um Mannitol, Sucrose, Glucose oder Lactose handelt.

6. Paclitaxel-Liposom-Zusammensetzung zur Verwendung in der Behandlung von Krebs nach den Ansprüchen 1 bis 4, bei welcher es sich bei der Aminosäure um Lysin, Threonin oder Methionin handeln kann.

7. Paclitaxel-Liposom-Zusammensetzung zur Verwendung in der Behandlung von Krebs nach den Ansprüchen 1 bis 4, bei welcher es sich bei dem Phosphatid um Eigelb-Lecithin oder Sojabohnen-Lecithin für die Injektion handelt.

8. Verfahren zur Herstellung der Paclitaxel-Liposom-Zusammensetzung zur Verwendung in der Behandlung von Krebs nach den Ansprüchen 1 bis 4, wobei das Verfahren die folgenden Schritte umfasst:
- 2-5 Gewichtsteile Paclitaxel, 20-200 Gewichtsteile Phosphatid, 2-30 Gewichtsteile Cholesterin werden nacheinander gerührt und in Isopropanol oder Ethanol gelöst, um eine klare Lösung zu erhalten;
- danach wird die Lösung in ein auf konstanter Temperatur gehaltenes Wasserbad, mit einer Temperatur von 50-60 °C, gestellt;
- nach Entfernen des Lösungsmittels mit einem Rotationsverdampfer unter vermindertem Druck bildet sich eine Membran;
- die wässrige Lösung von 0,3-4 Gewichtsteilen Aminosäuren und 10-75 Gewichtsteilen gefriergetrocknetem Hilfsstoff wird eingebracht;
- danach wird eine Hydratation, eine Beschallung oder eine Homogenisierung durchgeführt, um die Liposom-Größe von mehr als 0,1 µm zu erzielen;
- nach der Sterilfiltration wird die Ausbeute in Behälter unterverpackt und
- nach Gefriertrocknung wird Stickstoff-, Helium- oder Argongas zugeführt, um die Paclitaxel-Liposom-Zubereitung zu erhalten.

9. Verfahren zur Herstellung der Paclitaxel-Liposom-Zusammensetzung zur Verwendung in der Behandlung von Krebs nach den Ansprüchen 1 bis 4, wobei das Verfahren die folgenden Schritte umfasst:
- 3-5 Gewichtsteile Paclitaxel, 40-160 Gewichtsteile Phosphatid, 5-25 Gewichtsteile Cholesterin werden nacheinander gerührt und in Isopropanol oder Ethanol gelöst, um eine klare Lösung zu erhalten;
- danach wird die Lösung in ein auf konstanter Temperatur gehaltenes Wasserbad, mit einer Temperatur von 50-60 °C, gestellt;
- nach Entfernen des Lösungsmittels mit einem Rotationsverdampfer unter vermindertem Druck bildet sich eine Membran;
- die wässrige Lösung von 0,8-3 Gewichtsteilen Aminosäuren und 20-65 Gewichtsteilen gefriergetrocknetem Hilfsstoff wird eingebracht;
- danach wird eine Hydratation, eine Beschallung oder eine Homogenisierung durchgeführt;
- nach Sterilfiltration wird die Ausbeute in Behälter unterverpackt und
- nach Gefriertrocknung wird Stickstoff-, Helium- oder Argongas zugeführt, um die Paclitaxel-Liposom-Zubereitung zu erhalten.

10. Verfahren zur Herstellung der Paclitaxel-Liposom-Zusammensetzung zur Verwendung in der Behandlung von Krebs nach den Ansprüchen 1 bis 4, wobei das Verfahren die folgenden Schritte umfasst:
- 3-5 Gewichtsteile Paclitaxel, 60-120 Gewichtsteile Phosphatid, 8-20 Gewichtsteile Cholesterin werden nacheinander gerührt und in Isopropanol oder Ethanol gelöst, um eine klare Lösung zu erhalten;
- danach wird die Lösung in ein auf konstanter Temperatur gehaltenes Wasserbad, mit einer Temperatur von 50-60°C, gestellt;
- nach Entfernen des Lösungsmittels mit einem Rotationsverdampfer unter vermindertem Druck bildet sich eine Membran;
- die wässrige Lösung von 1,0-2 Gewichtsteilen Aminosäuren und 30-60 Gewichtsteilen gefriergetrocknetem Hilfsstoff wird eingebracht;
- danach wird eine Hydratation, eine Beschallung oder eine Homogenisierung durchgeführt;
- nach Sterilfiltration wird die Ausbeute in Behälter unterverpackt und
- nach Gefriertrocknung wird Stickstoff-, Helium- oder Argongas zugeführt, um die Paclitaxel-Liposom-Zubereitung zu erhalten.

11. Verfahren zur Herstellung einer Paclitaxel-Liposom-Zusammensetzung zur Verwendung in der Behandlung von Krebs nach den Ansprüchen 8 bis 10, bei welchem es sich bei dem gefriergetrockneten Hilfsstoff um Mannitol, Sucrose, Glucose oder Lactose handelt.

12. Verfahren zur Herstellung einer Paclitaxel-Liposom-Zusammensetzung zur Verwendung in der Behandlung von Krebs nach den Ansprüchen 8 bis 10, bei welchem es sich bei der Aminosäure um Lysin, Threonin oder Methionin handeln kann.

13. Verfahren zur Herstellung einer Paclitaxel-Liposom-Zusammensetzung zur Verwendung in der Behandlung von Krebs nach den Ansprüchen 8 bis 10, bei welchem es sich bei dem Phosphatid um Eigelb-Lecithin oder Sojabohnen-Lecithin für die Injektion handelt.

## Revendications

1. Composition liposomale de paclitaxel destinée à une utilisation dans le traitement du cancer, dans laquelle la composition est substantiellement constituée des matières suivantes en poids: 2 à 5 parties de paclitaxel, 20 à 200 parties de phosphatide, 2 à 30 parties de cholestérol, 0,3 à 4 parties d'acides aminés, 10 à 75 parties d'excipient lyophilisé.

2. Composition liposomale de paclitaxel destinée à une utilisation dans le traitement du cancer selon la revendication 1, dans laquelle la composition est essentiellement constituée des matières suivantes en poids :
3 à 5 parties de paclitaxel, 40 à 160 parties de phosphatide, 5 à 25 parties de cholestérol, 0,8 à 3 parties d'acides aminés, 20 à 65 parties d'excipient lyophilisé.

3. Composition liposomale de paclitaxel destinée à une utilisation dans le traitement du cancer selon la revendication 1, dans laquelle la composition est essentiellement constituée des matières suivantes en poids :
3 à 5 parties de paclitaxel, 60 à 120 parties de phosphatide, 8 à 20 parties de cholestérol, 1,0 à 2 parties d'acides aminés, 30 à 60 parties d'excipient lyophilisé.

4. Composition liposomale de paclitaxel destinée à une utilisation dans le traitement du cancer selon les revendications 1 à 3, dans laquelle la composition ne contient pas d'huile de ricin polyoxyéthylée.

5. Composition liposomale de paclitaxel destinée à une utilisation dans le traitement du cancer selon les revendications 1 à 4, dans laquelle ledit excipient lyophilisé est le mannitol, le saccharose, le glucose, ou le lactose.

6. Composition liposomale de paclitaxel destinée à une utilisation dans le traitement du cancer selon les revendications 1 à 4, dans laquelle ledit acide aminé peut être la lysine, la thréonine, ou la méthionine.

7. Composition liposomale de paclitaxel destinée à une utilisation dans le traitement du cancer selon les revendications 1 à 4, dans laquelle ledit phosphatide est la lécithine de jaune d'oeuf ou la lécithine de soja pour injection.

8. Procédé de préparation de la composition liposomale de paclitaxel destinée à une utilisation dans le traitement du cancer selon les revendications 1 à 4, dans lequel le procédé comprend les étapes suivantes consistant à :
- on agite et on dissout successivement 2 à 5 parties en poids de paclitaxel, 20 à 200 parties en poids de phosphatide, 2 à 30 parties en poids de cholestérol dans de l'isopropanol ou de l'éthanol, afin d'obtenir une solution transparente ;
- puis on place la solution dans un bain-marie à température constante ayant une température de 50 à 60°C ;
- après élimination du solvant à l'aide d'un évaporateur rotatif sous pression réduite, une membrane s'y forme ;
- on introduit la solution aqueuse de 0,3 à 4 parties en poids d'acides aminés et de 10 à 75 parties en poids d'excipient lyophilisé ;
- puis on réalise l'hydratation, la sonication ou l'homogénéisation afin d'obtenir une taille liposomale supérieure à 0,1 µm ;
- après stérilisation, filtration, le résultat est sous-conditionné dans des récipients et
- on ventile avec du gaz d'azote, d'hélium ou d'argon, après lyophilisation afin d'obtenir la préparation liposomale de paclitaxel.

9. Procédé de préparation de la composition liposomale de paclitaxel destinée à une utilisation dans le traitement du cancer selon les revendications 1 à 4, dans lequel le procédé comprend les étapes suivantes consistant à :
- on agite et on dissout successivement 3 à 5 parties en poids de paclitaxel, 40 à 160 parties en poids de phosphatide, 5 à 25 parties en poids de cholestérol dans de l'isopropanol ou de l'éthanol, afin d'obtenir une solution transparente ;
- puis on place la solution dans un bain-marie à température constante ayant une température de 50 à 60°C ;
- après élimination du solvant à l'aide d'un évaporateur rotatif sous pression réduite, une membrane s'y forme ;
- on introduit la solution aqueuse de 0,8 à 3 parties en poids d'acides aminés et de 20 à 65 parties en poids d'excipient lyophilisé ;
- puis on réalise l'hydratation, la sonication ou l'homogénéisation ;
- après stérilisation, filtration, le résultat est sous-conditionné dans des récipients et
- on ventile avec du gaz d'azote, d'hélium ou d'argon, après lyophilisation afin d'obtenir la préparation liposomale de paclitaxel.

10. Procédé de préparation de la composition liposomale de paclitaxel destinée à une utilisation dans le traitement du cancer selon les revendications 1 à 4, dans lequel le procédé comprend les étapes suivantes consistant à :
- on agite et on dissout successivement 3 à 5 parties en poids de paclitaxel, 60 à 120 parties en poids de phosphatide, 8 à 20 parties en poids de cholestérol dans de l'isopropanol ou de l'éthanol, afin d'obtenir une solution transparente ;
- puis on place la solution dans un bain-marie à température constante ayant une température de 50 à 60°C ;
- après élimination du solvant à l'aide d'un évaporateur rotatif sous pression réduite, une membrane s'y forme ;
- on introduit la solution aqueuse de 1,0 à 2 parties en poids d'acides aminés et de 30 à 60 parties en poids d'excipient lyophilisé ;
- puis on réalise l'hydratation, la sonication ou l'homogénéisation ;
- après stérilisation, filtration, le résultat est sous-conditionné dans des récipients et
- on ventile avec du gaz d'azote, d'hélium ou d'argon, après lyophilisation afin d'obtenir la préparation liposomale de paclitaxel.

11. Procédé de préparation de la composition liposomale de paclitaxel destinée à une utilisation dans le traitement du cancer selon les revendications 8 à 10, dans lequel ledit excipient lyophilisé est le mannitol, le saccharose, le glucose ou le lactose.

12. Procédé de préparation de la composition liposomale de paclitaxel destinée à une utilisation dans le traitement du cancer selon les revendications 8 à 10, dans lequel ledit acide aminé peut être la lysine, la thréonine ou la méthionine.

13. Procédé de préparation de la composition liposomale de paclitaxel destinée à une utilisation dans le traitement du cancer selon les revendications 8 à 10, dans lequel ledit phosphatide est la lécithine de jaune d'oeuf ou la lécithine de soja pour injection.
